# EUROPÄISCHE PATENTSCHRIFT

(11) **EP 2 667 841 B1**
(45) Veröffentlichungstag und Bekanntmachung des Hinweises auf die Patenterteilung: **25.11.2015**
(21) Anmeldenummer: 11799440.0
(22) Anmeldetag: 21.12.2011
(51) Int. Cl.: A61K 8/02, A61K 8/89, A61Q 1/02, A61Q 1/06, A61Q 1/10, A61Q 1/12, A61Q 5/02, A61Q 5/06, A61Q 5/12, A61Q 15/00, A61Q 17/04, A61Q 19/00, A61Q 19/10, C08G 77/38, C08L 51/08, A61K 8/81, A61K 8/895, C08F 290/08, C08G 77/20, C08F 290/06, C08F 299/08

(54) **VERWENDUNG VON SILICONMETHACRYLAT-PARTIKELN IN KOSMETISCHEN FORMULIERUNGEN**
USE OF SILICONE METHACRYLATE PARTICLES IN COSMETIC FORMULATIONS
UTILISATION DE PARTICULES DE METHACRYLATE DE SILICONE DANS DES FORMULATIONS COSMETIQUES

(30) Priorität: 25.01.2011 DE 102011003090
(43) Veröffentlichungstag der Anmeldung: 04.12.2013
(73) Patentinhaber: Evonik Degussa GmbH, 45128 Essen (DE)
(72) Erfinder: JHA, Brajesh Kumar, Midlothian Virginia 23112 (US); MEYER, Juergen, 45134 Essen (DE); NAUMANN, Matthias, 22159 Hamburg (DE); VENZMER, Joachim, 45239 Essen (DE); WIECHERS, Susann, 45239 Essen (DE)
(86) Internationale Anmeldenummer: PCT/EP2011/073544
(87) Internationale Veröffentlichungsnummer: WO 2012/100884

(56) Entgegenhaltungen:
- EP-A1- 2 067 811
- WO-A2-2009/133765

## Beschreibung

Die vorliegende Erfindung betrifft kosmetische Zusammensetzungen enthaltend Feststoffpartikel, dadurch gekennzeichnet, dass als Feststoffpartikel Siliconmethacrylat-Partikel verwendet werden, die erhältlich sind durch die Schritte a) Erzeugen einer Emulsion aus Wasser und einer organischen Phase, wobei die organische Phase, speziell, end- und/oder seitenständig mit Methacrylat-Gruppen modifizierte Organopolysiloxane oder deren Gemische enthält, unter Zugabe zumindest eines Emulgators und optional eines oder mehrerer Coemulgatoren, wobei die organische Phase die innere Phase der Emulsion bildet, und Auspolymerisieren der inneren Phase in Gegenwart eines Radikalstarters, welcher der äußeren Phase (wässrigen Phase) in einer Konzentration von 0,1 bis 40 Gew.-% bezogen auf die innere Phase zugefügt wird.

Es ist bekannt hydrophobe bzw. hydrophobierte Partikel oder Siliconharz-Partikel in kosmetischen, insbesondere Make-up Formulierungen zu verwenden, um die Haltbarkeit des Make-ups auf der Haut zu verbessern. Dazu werden häufig Silicon-Gummi-Pulver oder Pulver von Silicon-Elastomeren eingesetzt.

In EP 0 834 305 werden gelartige kosmetische Hautbehandlungszusammensetzungen beschrieben, die kugelförmige Pulver von Organopolysiloxanelastomeren mit einer mittleren Partikelgröße von 1 bis 15 µm enthalten. Als weitere Partikel werden insbesondere hydrophobierte bzw. mit Silicon behandelte anorganische Pulver zugesetzt.

In EP 0 765 656 werden kosmetische Wasser-in-Öi Emulsionen als kosmetische Zusammensetzungen beschrieben, die Pulver von sphärischen, elastomeren Organopolysiloxan-Partikeln enthalten. Neben den elastomeren (verformbaren) Partikeln weisen diese Zusammensetzungen hydrophobierte Kieselsäure Partikel auf.

In FR 2682534 werden Hautkosmetika beschrieben, die zwei verschiedene Partikelfraktionen aufweisen, wobei die eine Fraktion aus nicht verformbaren Partikeln, vorzugsweise Glasperlen und die andere aus verformbaren, also elastischen Partikeln besteht.

Den genannten Dokumenten ist gemein, dass durch die Verwendung der Elastomerpartikel ein angenehmes (weiches), pudriges Hautgefühl erreicht werden soll.

Nachteilig an der Verwendung elastomerer Partikel ist häufig der Gehalt an in den Siliconelastomerpartikeln enthaltenem Siliconöl, das in gewissen kosmetischen Formulierungen unerwünscht ist und bei Austritt die Zusammensetzung und Stabilität der Formulierung negativ beeinflussen kann, sowie die mit der Herstellung verbundenen hohen Kosten und der eingeschränkte Formulierungsspielraum.

Aufgabe der vorliegenden Erfindung bestand in der Bereitstellung kosmetischer Zusammensetzungen die Partikel enthalten, die einen oder mehrere der genannten Nachteile der elastomeren Partikel nicht aufweisen.

Überraschenderweise wurde gefunden, dass die Verwendung von nachfolgend beschriebenen Siliconmethacrylat-Partikeln in kosmetischen Zusammensetzungen Hautkosmetika zugänglich macht, die ein angenehmes, samtig-pudriges Hautgefühl vermitteln, das dem Hautgefühl von typischen Silicon-Elastomer-haltigen Verbindungen entspricht, ohne dass die eingesetzten Partikel Siliconelastomerpartikel sind bzw. deren Nachteile aufweisen.

Gegenstand der Erfindung ist daher eine Zusammensetzung, wie in Anspruch 1 definiert, enthaltend Feststoffpartikel, welche dadurch gekennzeichnet ist, dass als Feststoffpartikel Siliconmethacrylat-Partikel enthalten sind, die erhältlich sind durch die Schritte: a) Erzeugen einer Emulsion aus Wasser und einer organischen Phase, wobei die organische Phase, end- und/oder seitenständig mit Methacrylat-Gruppen modifizierte Organopolysiloxane nach der allgemeinen Formel (I) oder deren Gemische, mit der Maßgabe, dass wenn b und c = 0 sind, R³ nicht aus der gleichen Gruppe wie R¹ ausgewählt sein darf, enthält, unter Zugabe zumindest eines Emulgators und optional eines oder mehrerer Coemulgatoren, wobei die organische Phase die innere Phase der Emulsion bildet, und b) Auspolymerisieren der inneren Phase in Gegenwart eines Radikalstarters, welcher der äußeren Phase (wässrigen Phase) in einer Konzentration von 0,1 bis 40 Gew.-% bezogen auf die innere Phase zugefügt wird.

Im Vergleich mit den in DE 10 2007 058 713 beschriebenen Siliconacrylat-Partikeln, weisen die erfindungsgemäß eingesetzten Siliconmethacrylat-Partikel den Vorteil auf, dass mit ihnen hergestellte Kosmetika eine deutlich höhere Lagerstabilität aufweisen. Die Siliconacrylatpartikel weisen bereits im unverarbeiteten Zustand nach mehreren Wochen Lagerung bei Raumtemperatur einen deutlich wahrnehmbaren Geruch nach Acrylsäure auf, die durch Hydrolyse mit der Luftfeuchtigkeit entsteht. In einer Wasser enthaltenden Formulierung tritt dieser Effekt ebenfalls auf, was in den Beispielen beschrieben wird.

Die erfindungsgemäße Verwendung der Siliconmethacrylat-Partikel zur Herstellung von kosmetischen Zusammensetzungen (Emulsionen) hat außerdem den Vorteil, dass diese einen stabilisierenden Effekt aufweisen. So können erfindungsgemäße Zusammensetzungen, die die Siliconmethacrylat-Partikel enthalten, eine bessere Lagerstabilität aufweisen aber auch eine höhere Stabilität beim Durchfahren von Gefrier-Tau-Zyklen aufweisen.

Auf Grund des stabilisierenden Effekts der verwendeten Siliconmethacrylat-Partikel können mit diesen stabile kosmetische Zusammensetzungen (Emulsionen) hergestellt werden, ohne dass die sonst notwendige Menge an Emulgator verwendet werden muss. Unter stabilen Emulsionen werden im Rahmen der vorliegenden Erfindung solche Emulsionen aus Wasser und Öl verstanden, die auch nach einer Lagerung von 3 Monaten bei 25 °C keine mit bloßem Auge sichtbare Separation in Öl- und Wasserphase zeigen.

Die erfindungsgemäßen Zusammensetzungen und die erfindungsgemäße Verwendung von Siliconmethacrylat-Partikeln werden nachfolgend beispielhaft beschrieben, ohne dass die Erfindung auf diese beispielhaften Ausführungsformen beschränkt sein soll.

Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z. B. organomodifizierte Polysiloxane, beschrieben, die verschiedene Einheiten mehrfach aufweisen können, so können diese statistisch verteilt (statistisches Oligomer oder Polymer) oder geordnet (Blockoligomer oder Blockpolymer) in diesen Verbindungen vorkommen. Wenn nicht anders angegeben, handelt es sich bei Mittelwertangaben um Zahlenmittel und bei Prozent-Angaben um Angaben in Massen-%.

Erfindungsgemäße kosmetische Zusammensetzungen enthaltend Feststoffpartikel zeichnen sich dadurch aus, dass die Zusammensetzung als Feststoffpartikel zumindest Siliconmethacrylat-Partikel aufweist, die erhältlich sind durch die Schritte a) Erzeugen einer Emulsion aus Wasser und einer organischen Phase, wobei die organische Phase, end- und/oder seitenständig mit Methacrylat-Gruppen modifizierte Organopolysiloxane nach der allgemeinen Formel (I) oder deren Gemische 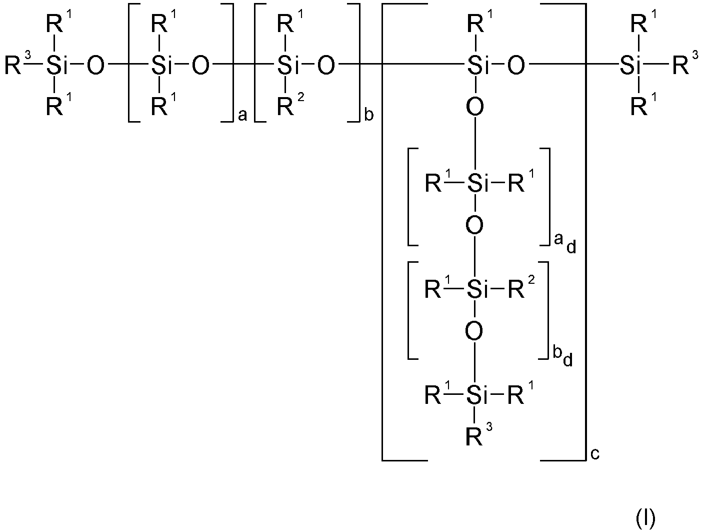 mit
R¹ = gleiche oder verschiedene Reste, ausgewählt aus linearen oder verzweigten, gesättigten, ein- oder mehrfach ungesättigten, gegebenenfalls eine oder mehrere Ether- oder Esterbrücken enthaltende lineare, cyclische oder verzweigte Alkyl-, Alkoxy-, Polyalkoxy-, Hydroxyalkyl-, Hydroxyalkoxy-, Alkenyl-, Aryl-, Aryloxy-, Hydroxyaryl-, Hydroxyaryloxy-, Alkaryl-, Alkaryloxy-, Hydroxyalkaryl-, Hydroxyalkaryloxy-, Aralkyl-, Aralkoxy-, Hydroxyaralkyl- oder Hydroxyaralkoxy-Resten mit 1 bis 20 C-Atomen, vorzugsweise gleiche oder verschiedene Reste, ausgewählt aus linearen oder verzweigten, gesättigten, ein- oder mehrfach ungesättigten, gegebenenfalls eine oder mehrere Ether- oder Esterbrücken enthaltende lineare, cyclische oder verzweigte Alkyl-, Aryl-, Alkaryl- oder Aralkylresten mit 1 bis 20 C-Atomen, bevorzugt Methyl-Rest,
R² = gleiche oder verschiedene, an das Si-Atom über eine Si-C-Verknüpfung gebundene, zweiwertige, Kohlenwasserstoffreste, mit 1 bis 20 C-Atomen, an welche mindestens eine Methacrylsäure-Einheiten über eine Esterbindung angebunden ist,
R³ = gleiche oder verschiedene Reste R¹ oder R², vorzugsweise R²,
a = 50 bis 1.000, vorzugsweise 100 bis 210, besonders bevorzugt 140 bis 190,
b = 0 bis 15, vorzugsweise 5 bis 10,
c = 0 bis 5, vorzugsweise 0,
a_{d} = 0 bis 1.000,
b_{d} = 0 bis 15,
wobei der Index d für c > 0 eine ganze Zahl > 0 ist, mit der Maßgabe, dass wenn b und c = 0 sind, R³ nicht aus der gleichen Gruppe wie R¹ ausgewählt sein darf, enthält, unter Zugabe zumindest eines Emulgators, vorzugsweise eines Festkörperemulgators (partikulären Emulgators), und optional eines oder mehrerer Coemulgatoren, wobei die organische Phase die innere Phase der Emulsion bildet, und b) Auspolymerisieren der inneren Phase in Gegenwart eines Radikalstarters, welcher der äußeren Phase (wässrigen Phase) in einer Konzentration von vorzugsweise von 0,1 bis 40 Gew.-%, bevorzugt 0,5 bis 25 Gew.-% und besonders bevorzugt 10 bis 15 Gew.-%, bezogen auf die innere Phase zugefügt wird. Das Auspolymerisieren erfolgt also bevorzugt in Form einer Suspensionspolymerisation.

Die Zahlenwerte für a, b und c stellen vorzugsweise statistische (Zahlen-)Mittelwerte dar. Der Index d ist ein ganzzahliger Indexterm (Laufvariable).

Die erfindungsgemäß eingesetzten Siliconmethacrylat-Partikel sind vorzugsweise nicht elastisch bzw. im Wesentlichen (größer 90 Massen-%) aus nicht-elastischem Material aufgebaut. Unter nicht-elastischem Material wird im Rahmen der vorliegenden Erfindung eine Material verstanden, welches in Masse polymerisiert bei einer Prüfung in Anlehnung an DIN 53 504 nicht ohne zu reißen zu 100 %, vorzugsweise nicht zu 50 % und besonders bevorzugt nicht zu 25 % gedehnt werden kann.

In der erfindungsgemäßen kosmetischen Zusammensetzung werden vorzugsweise Siliconmethacrylat-Partikel eingesetzt, die ein Maximum der Partikelgrößenverteilung im Bereich von 1 bis 50 µm, bevorzugt 5 bis 20 µm aufweisen. Durch die Verwendung der Siliconmethacrylat-Partikel im genannten Partikelgrößebereich wird ein angenehmes samt-seidiges, pudriges Hautgefühl erreicht. Liegt das Maximum der Partikelgrößenverteilung bei größeren Partikelgrößen erhält man ein eher abrasives Hautgefühl, kleinere Partikelgrößen haben entweder keine sensorische Wirkung oder erzeugen ein eher stumpfes Hautgefühl.

Die Partikelgrößenverteilung der trockenen Siliconmethacrylatpartikel kann mit einem Meßsystem wie zum Beispiel der Firma Sympatec bestimmt werden, bestehend aus den Modulen VIBRI, RODOS und HELOS. Die Partikel werden durch Vibration vordispergiert und mit Druckluft, vorzugsweise mit einem Überdruck von 2 bis 3 bar, dispergiert und durch eine Meßkammer geblasen. Dort wird mittels Laserbeugung die Partikelgrößenverteilung bestimmt. Die Auswertung erfolgt mit der dazugehörigen WINDOX-Software.

Bevorzugte erfindungsgemäße kosmetische Zusammensetzungen sind solche, die einen Anteil der Siliconmethacrylat-Partikel an der Gesamtzusammensetzung von 0,01 bis 80 Gew.-%, vorzugsweise von 0,1 bis 10 Gew.-% aufweisen.

Die erfindungsgemäßen Zusammensetzungen können als weitere Komponenten solche aufweisen, wie sie in kosmetische Zusammensetzungen üblich sind. So können die erfindungsgemäßen Zusammensetzungen zum Beispiel eine oder mehrere zusätzliche Komponenten, ausgewählt aus der Gruppe der
Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel,
enthalten.

Substanzen, die als beispielhafte Vertreter der einzelnen Gruppen vorhanden sein können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Besonders bevorzugt weisen die erfindungsgemäßen Zusammensetzungen eine oder mehrere der nachfolgend aufgeführten Komponenten auf:
Antimikrobielle und/oder konservierende Stoffe, wie Triclosan und Triclocarban und Hexachlorophen, Komplexbildner wie beispielsweise EDTA (Säure und Salze), Zitronensäure und Etidronsäure sowie deren Salze, UV-Absorber, wie beispielsweise Derivate des Benzophenons, des Benzotriazols, Zimtsäureester oder partikuläre UV-Absorber wie beispielsweise ZnO oder TiO₂, Farbstoffe und Färbemittel, Pigmente, Sprühhilfsmittel, Netzmittel, Vitamine, Wuchsstoffe, Hormone sowie Duftstoffe.

Es kann vorteilhaft sein, wenn die erfindungsgemäße Zusammensetzung von 0,1 bis 20 Gew.-% bezogen auf die Gesamtzusammensetzung an Komponenten aufweist, die UV-Strahlung absorbieren oder ausfiltern. Solche Substanzen können z. B. die oben genannten UV-Absorber sein.

Die erfindungsgemäßen kosmetischen Zusammensetzungen (Emulsionen) können weitere Feststoffe (kosmetische Partikel), die von den genannten Siliconmethacrylat-Partikeln verschieden sind, insbesondere Pigmente, wie Titandioxid, Zinkoxid oder Eisenoxide, Viskositätsregulatoren, wie pyrogene oder gefällte Kieselsäure oder andere Partikel enthalten. Bevorzugte erfindungsgemäße Zusammensetzungen weisen von 0,1 bis 15 Gew.-% an Partikeln auf, die von den genannten Siliconmethacrylat-Partikeln verschieden sind. Die erfindungsgemäß verwendeten Siliconmethacrylatpartikel ermöglichen es dabei, das stumpfe Hautgefühl dieser anderen Pigmente (kosmetische Partikel) in den Zusammensetzungen durch ein pudrig samtiges Hautgefühl zu überdecken. Besonders bevorzugt ist somit der Einsatz der Siliconmethacrylatpartikel in Sonnenschutzprodukten, Make-Up Produkten, Lippenstiften und anderen kosmetischen Produkten für dekorative Anwendungen.

Die weiteren eingesetzten Feststoffe (Partikel), die von den genannten Siliconmethacrylat-Partikeln verschieden sind, haben vorzugsweise eine Teilchengröße von 5 bis 50 µm und können z.B. zur Erzielung eines verbesserten Hautgefühls, einer Mattierung oder zur optischen Faltenreduzierung ("soft focus effect") eingesetzt werden. Typische Partikelmaterialien sind z.B. PMMA, PS, PE, PP, Nylon, insbesondere Nylon-12 und Nylon-6, Siliconpartikel bzw. Siliconelastomere, Stärke, Talkum, Mica und Bornitrid. Die eingesetzten Partikel können dabei je nach Eigenschaftsprofil sowohl eine kompakte als auch poröse Struktur haben. Als weitere Feststoffe (kosmetische Partikel) im Sinne der Erfindung können in den erfindungsgemäßen Zusammensetzungen auch Siliconelastomergele enthalten sein. Bei den Siliconelastomergelen handelt es sich vorzugsweise um Siliconelastomere, die direkt in einem Trägeröl hergestellt wurden. Bei den Trägerölen handelt es sich vorzugsweise um typische kosmetische Öle, wie beispielsweise zyklische oder lineare Siliconöle, Mineralöle, Esteröle, Etheröle oder Triglyceride. Typische Siliconelastomergele sind beispielsweise Dow Corning 9040 Silicone Elastomer Blend und Dow Corning 9041 Silicone Elastomer Blend (Dow Corning) oder die Produkte KSG-15 oder KSG-18 (Shin Etsu).

Es kann vorteilhaft sein, wenn die erfindungsgemäßen Zusammensetzungen von 0,1 bis 20 Gew.-% an siliziumorganischen Verbindungen aufweisen. Die siliziumorganischen Verbindungen können dabei z. B. als Emulgatoren oder als Emollient in der erfindungsgemäßen Zusammensetzung vorhanden sein. Geeignete siliziumorganische Verbindungen sind beispielsweise cyclische oder lineare Alkylsiloxane, insbesondere Methylsiloxane, wie z. B. Dimethicon, D4- oder D5-Cyclen, die vorzugsweise als Emollient in der erfindungsgemäßen Zusammensetzung vorhanden sind, oder organomodifizierte Siloxane, insbesondere polyethermodifizierte Polysiloxane. Solche organomodifizierte Siloxane sind vorzugsweise als Emulgatoren in der erfindungsgemäßen Zusammensetzung enthalten.

Weitere spezielle siliziumorganische Verbindungen sind insbesondere kosmetische Emulgatoren für O/W und W/O Emulsionen, dabei handelt es sich vorzugsweise um: Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15, weiterhin alkoxyilierte Polysiloxan-Polyether-Copolymere wie z.B. Methoxy PEG/PPG-25/4 Dimethicone. Weiterhin auch endständig modifizierte Siliconpolyether wie etwa Bis-PEG/PPG-14/14-Dimethicone; Polysiloxan-Polyalkyl-Polyether-Copolymere bzw. entspre-chende Derivate, wie z.B. Lauryl oder Cetyl Dimethicone Copolyole, insbesondere Cetyl PEG/PPG-10/1 Dimethicone (ABIL^{®} EM 90 (Evonik Goldschmidt)); Polyethersiloxane, die sowohl kamm- und endständige Modifikation enthalten, wie z.B. Bis- PEG/PPG-20/5 PEG/PPG-20/5 Dimethicone oder Bis-PEG/PPG-16/16 PEG/PPG-16/16 Dimethicone; PEG-freie Siliconemulgatoren, wie z.B. Lauryl Glyceryl Dimethicone, Lauryl Polyglyceryl-3 Polydimethylsiloxyethyl Dimethicone, Polyglyceryl-3 Disiloxane Dimethicone oder Amodimethicone Glycerocarbamat.

Bei den erfindungsgemäßen, Siliconmethacrylatpartikel enthaltenden kosmetischen Zusammensetzungen handelt es sich bevorzugt um kosmetische Emulsionen. Diese können sowohl vom Typ Wasser-in-Öl (W/O) als auch vom Typ Öl-in-Wasser (O/W) sein. Bevorzugt kann dabei die Ölphase dieser Emulsionen ein oder mehrere Siliconöle enthalten, teilweise können diese Emulsionen ausschließlich Siliconöle als Öle enthalten. In diesem Falle kann man diese erfindungsgemäßen Emulsionen auch als W/Si- oder Si/W-Emulsionen bezeichnen. Besonders bevorzugte Siliconöle sind dabei flüssige Cyclo- oder Dimethicone mit einer Viskosität von 1 bis 200 mPas.

Besonders bevorzugt erfindungsgemäße Zusammensetzungen weisen jeweils bezogen auf die Gesamtzusammensetzung von 0,1 bis 20 Gew.-% an Komponenten, die UV-Strahlung absorbieren oder ausfiltern, von 0,1 bis 15 Gew.-% an Partikeln, die von den genannten Siliconmethacrylat-Partikeln verschieden sind, und von 0,1 bis 20 Gew.-% an siliziumorganischen Verbindungen auf.

Das Verfahren zur Herstellung der Siliconmethacrylat-Partikel kann prinzipiell, wie in DE 10 2007 058 713 allgemein für Silicon(meth-)crylat-Partikel beschrieben, auf welche vollumfänglich Bezug genommen wird, durchgeführt werden.

Als Emulgatoren zur Herstellung der Siliconmethacrylat-Partikel können alle üblichen Emulgatoren eingesetzt werden. Dabei kann es sich um anionische, kationische oder nichtionische oberflächenaktive Substanzen handeln.

Typische Emulgatoren sind z. B. Alkylsulfate, vorzugsweise mit einer Kettenlänge von 10 bis 18 C-Atomen, Alkyl- und Arylethersulfate, vorzugsweise mit 10 bis 24 C-Atomen im hydrophoben Rest und mit bevorzugt bis zu 40 Ethylenoxid- oder Propylenoxid-Einheiten, Alkyl- und alkylarylsulfonate mit vorzugsweise 10 bis 24 C-Atomen, Alkyldiphenyloxiddisulfonate, Ölsäuresulfonate, Ester und Halbester der Sulfobernsteinsäure mit einwertigen Alkoholen oder Alkylphenolen, Alkyl- und Alkenylcarboxylate mit vorzugsweise einer Kettenlänge von 10 bis 18 C-Atomen, Alkylpolyglykolether und Alkylarylpolyglykolether mit vorzugsweise jeweils 4 bis 40 Ethylenoxideinheiten, Alkyl- und Alkenylalkohole mit vorzugsweise 12 bis 20 C-Atomen, ethoxylierte Alkyl- und Alkenylalkohole mit vorzugsweise 12 bis 20 C-Atomen und ethoxylierte Alkylphenole. Für kosmetische Anwendungen eignen sich insbesondere Emulgatorsysteme, die üblicherweise zur Emulgierung von Siliconölen dienen, wie sie beispielsweise von der Evonik Goldschmidt GmbH unter den Namen ABIL^{®} EM 90, ABIL^{®} EM 97 oder ABIL^{®} Care XL 80 angeboten werden. Insbesondere können aus kosmetischen Anwendungen bekannte Emulgatoren und Tenside eingesetzt werden, wie sie etwa in DE 10 2005 011785 A1 aufgeführt sind.

Es kann vorteilhaft sein, wenn in Schritt a) eine festkörperstabilisierte Emulsion erzeugt wird. Dazu können als partikuläre Emulgatoren Partikel eingesetzt werden, die besonders bevorzugt ausgewählt sind aus der Gruppe der Metalloxide, Mischoxide, Nitride, Hydroxide, Carbonate, Silikate, Siliconharze, Silicone und/oder organischen Polymere, die vorzugsweise zumindest (teil-)hydrophobiert sind, z. B. mit mindestens einer Verbindung aus der Gruppe der Silane, Siloxane, quaternären Ammoniumverbindungen, kationischen Polymere und Fettsäuren oder deren Anionen. Besonders bevorzugt eingesetzte partikuläre Emulgatoren sind beispielsweise colloidale Silica-Partikel, wie sie unter dem Handelsnamen LUDOX® von Grace Davidson erhältlich sind.

Die partikulären Emulgatoren können in dem erfindungsgemäßen Verfahren als solche oder in Form von Dispersionen oder Solen, insbesondere wässrigen Dispersionen oder Solen eingesetzt werden. Es kann vorteilhaft sein, wenn partikuläre Emulgatoren eingesetzt werden, die vorzugsweise eine mittlere Primärpartikelgröße in allen Dimensionen von größer 0,1 bis 1 µm aufweisen. Die Bestimmung der Primärpartikelgröße kann z.B. durch optische Auswertung einer durch Transmissionselektronenmikroskopie erstellten Aufnahme bestimmt werden.

Insbesondere bei der Verwendung von partikulären Emulgatoren kann es vorteilhaft sein, wenn in Schritt a) des erfindungsgemäßen Verfahrens die Herstellung der Emulsion unter Zugabe eines oder mehrerer Coemulgatoren durchgeführt wird. Als Coemulgatoren können in dem erfindungsgemäßen Verfahren insbesondere solche Verbindungen eingesetzt werden, die mit den Festkörperemulgator-Partikeln in Wechselwirkung treten, vorzugsweise solche, die zu hydrophobierenden Festkörperemulgator-Partikel aufziehen. In dem erfindungsgemäßen Verfahren können als Coemulgatoren insbesondere Verbindungen ausgewählt aus der Gruppe der kationischen Tenside eingesetzt werden. Als kationische Coemulgatoren können insbesondere kationische Ammoniumverbindungen eingesetzt werden. Solche Verbindungen sind z. B. unter den Handelsnamen VARISOFT® 470 P, VARISOFT® TC-90, VARISOFT® 110, VARISOFT® TA-100, ADOGEN® 442-100 P, ADOGEN® 432, ADOGEN® 470, ADOGEN® 471, ADOGEN® 464, VARIQUAT® K 300, VARIQUAT® B 343, VARIQUAT® 80 ME, REWOQUAT® 3690, REWOQUAT® WE 15, REWOQUAT® WE18, REWOQUAT® WE 28 oder REWOQUAT® CR 3099 bei der Evonik Goldschmidt GmbH erhältlich. Bevorzugt wird in dem erfindungsgemäßen Verfahren VARISOFT® TA-100 oder VARISOFT® PATC (beide erhältlich bei der Evonik Goldschmidt GmbH), besonders bevorzugt VARISOFT® PATC als kationischer Coemulgator eingesetzt. Ganz besonders bevorzugt werden in dem erfindungsgemäßen Verfahren solche Siliconmethacrylat-Partikel eingesetzt, bei deren Herstellung kein Cetyltrimethylammoniumbromid eingesetzt wird/wurde.

In dem Verfahren werden als Siliconmethacrylate der Formel (I) vorzugsweise solche eingesetzt, bei denen mehr als 70 mol-%, besonders bevorzugt mehr als 95 %, ganz besonders bevorzugt alle der Reste R¹ in Formel (I) Methylgruppen sind.

Die Reste R² in der allgemeinen Formel (I) sind vorzugsweise ausgewählt aus der Gruppe der Reste 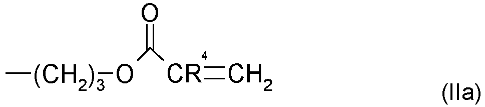 und 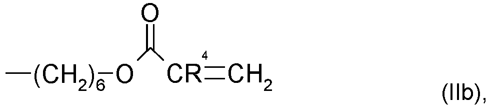 wobei R⁴ eine Methylgruppe ist.

Besonders vorteilhaft kann es sein, wenn solche Siliconmethacrylat-Partikel eingesetzt werden, bei deren Herstellung Siliconmethacrylate der Formel (I) eingesetzt werden, bei denen a einen Wert von 100 bis 210, bevorzugt von 140 bis 190 und b einen Wert von 3 bis 9 einnimmt und c = 0 ist, wie sie in der Patentschrift DE 3810140 beschrieben sind.

Im Verfahren zur Herstellung der Siliconmethacrylat-Partikel können die Siliconmethacrylate der Formel (I) einzeln oder als Mischungen, insbesondere als statistische Mischungen eingesetzt werden. Vorzugsweise werden in dem erfindungsgemäßen Verfahren Mischungen von Siliconmethacrylaten der Formel (I) eingesetzt, in denen die Siliconmethacrylate sich bezüglich ihrer Struktur und/oder ihrem Molekulargewicht unterscheiden.

Für weitere Details zum Herstellungsverfahren der Siliconmethacrylat-Partikel sowie für bevorzugte Ausführungsformen des Verfahrens zur Herstellung von Siliconmethacrylatpartikel wird explizit auf die Beschreibung der DE 10 2007 058 713 A1 verwiesen.

Die Herstellung der Zusammensetzungen/Dispersionen unter Verwendung der Siliconmethacrylat-Partikel kann mittels der üblichen Methoden entsprechend dem Stand der Technik erfolgen, jedoch können auch die nach der Polymerisation gemäß Schritt b) des Herstellungsverfahrens der Partikel und Waschen mit Alkoholen und/oder Wasser entstandenen Partikel ohne vorheriges Trocknen zu beispielsweise wässrigen Dispersionen weiterverabeitet werden. Dies ist auch möglich, wenn beispielsweise eine gewünschte Oberflächenmodifikation direkt aus wässriger oder alkoholischer Phase erfolgen kann, was sich günstig auf die Verfahrensökonomie auswirkt.

Die erfindungsgemäßen Siliconmethacrylatpartikel können bei Herstellung kosmetischer Emulsionen entweder direkt in die heiße oder kalte Ölphase eingearbeitet werden, oder sie können nach Kombination von Wasser-und Ölphase zu einer W/O oder O/W Emulsion, bevorzugt bei Temperaturen kleiner 50°C, in die bereits gebildete Emulsion eingerührt werden.

Die erfindungsgemäßen kosmetischen Zusammensetzungen sind vorzugsweise ein Hautpflegemittel, ein Make-up oder ein Sonnenschutzmittel.

Außerdem Gegenstand der vorliegenden Erfindung ist die Verwendung von Siliconmethacrylat-Partikel, die erhältlich sind durch das oben beschriebene Verfahren umfassend die Schritte a) und b), zur Herstellung kosmetischer Zusammensetzungen enthaltend Feststoffpartikel. Vorzugsweise werden die oben als bevorzugt beschriebenen bzw. die mit dem bevorzugten Verfahren hergestellten Siliconmethacrylat-Partikel verwendet. Insbesondere werden solche Siliconmethacrylat-Partikel verwendet, die ein Maximum der Partikelgrößenverteilung im Bereich von 1 bis 20 µm aufweisen, und/oder, die durch Erzeugen einer festkörperstabilisierte Emulsion in Schritt a) erhalten wurden, wobei als Emulgatoren partikuläre Emulgatoren, die ausgewählt sind aus der Gruppe der Metalloxide, Mischoxide, Nitride, Hydroxide, Carbonate, Silikate, Siliconharze, Silicone und/oder organischen Polymere, die zumindest (teil-)hydrophobiert sind mit mindestens einer Verbindung aus der Gruppe der Silane, Siloxane, quaternären Ammoniumverbindungen, kationischen Polymere und Fettsäuren oder deren Anionen, und/oder bei denen mehr als 95 mol-% der Reste R¹ in Formel (I) Methylgruppen sind, und/oder bei denen R² in der allgemeinen Formel(I) aus der Gruppe der Reste 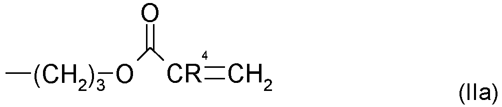 und 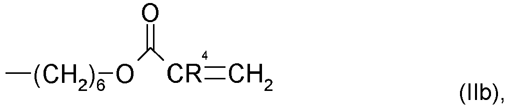 ausgewählt ist,
wobei R⁴ eine Methylgruppe ist, und/oder bei deren Herstellung Siliconmethacrylate der Formel (I) eingesetzt werden, bei denen a einen Wert von 150 bis 210 und b einen Wert von 3 bis 9 einnimmt und c = 0 ist.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll. Sind in den nachfolgenden Beispielen Prozentangaben genannt, so handelt es sich, wenn nicht anders angegeben, um Angaben in Massen-Prozent.

Die nachfolgend aufgeführten Formulierungsbeispiele sind in der Mehrzahl Emulsionen vom Typ Öl-in-Wasser (O/W) oder Wasser-in-Öl (W/O). Diese können nach üblichen, dem Fachmann bekannten, Methoden unter Verwendung typischer Rühraggregate hergestellt werden. Vorzugsweise werden W/O-Emulsionen durch langsames Einrühren der Wasserphase (B) in die Ölphase (A) mit nachfolgender Homogenisierung hergestellt. Bei den beschriebenen O/W Emulsionen werden vorzugsweise Öl- und Wasserphase ohne Rühren zusammengeführt und anschließend homogenisiert. Dies kann in einem Kalt-Kalt-Prozess geschehen (Beispiel 3) oder in einem Heiß-Heiß-Prozess, bei dem die Homogenisierung bei ca. 70°C stattfindet (Beispiel 2). Die erfindungsgemäßen Siliconmethacrylatpartikel können dabei prinzipiell in jeder Stufe des Prozesses eingearbeitet werden. In den meisten Beispielformulierungen erfolgte entweder die Einarbeitung in die fertige Emulsion bei Temperaturen < 40°C oder die Siliconmethacrylatpartikel wurden direkt zu Beginn der Emulisonsherstellung zusammen mit der Ölphase vorgelegt.

Der zur Auswertung der Emulsionsvergleichsbeispiele verwendeten Nomenklatur zum Thema "Stabilität" liegen folgende Anforderungen zugrunde. Ist die Stabilität mit "gut" bewertet, so bedeutet das, dass eine solche Emulsion mindestens einen Monat bei Raumtemperatur, -5°C und 40°C stabil ist. "Stabil" bedeutet dabei, dass keinerlei Öl- oder Wasserabscheidung auftritt, dass das Erscheinungsbild der Emulsion homogen bleibt und dass in der Emulsion keine nennenswerten Veränderungen von Viskosität, Farbe oder Geruch auftreten.

Das Hautgefühl der in den folgenden Beispielen beschriebenen kosmetischen Formulierungen wurde durch ein sogenanntes Panel bestimmt. Mindestens fünf Personen verglichen die sensorischen Eigenschaften der kosmetischen Formulierungen und der jeweiligen Vergleichsformulierung ohne die Zusammensetzung zu kennen. Es werden die Eigenschaften aufgeführt, die die Mehrheit der Personen bevorzugt beschrieben hat.

### Beispiel 1: Partikel aus Siliconmethacrylat

588, 5 g Dikaliumhydrogenphosphat wurden in 550 g demineralisiertem Wasser gelöst. 2600 g demineralisiertes Wasser und 115 g Ludox® SM wurden vermischt und auf pH=7 eingestellt. Unter Rühren wurden 1100 g Siliconmethacrylat (beschrieben in der Patentschrift DE 3810140) zugefügt und mehrere Minuten voremulgiert. Anschließend wurden unter Rühren 27,5 g einer 5 %-igen wässrigen Lösung von VARISOFT® PATC hinzugefügt und nochmals einige Minuten voremulgiert. Anschließend wurde das so entstandene Gemisch mit einem Homogenisator des Typs Microfluidizer der Firma Microfludics mit einer Interaktionskammer mit 200 µm Durchmesser bei 300 bar homogenisiert.

Zur Polymerisation wurde die Emulsion in einen Rundkolben gegeben und unter Rühren mit Stickstoff gespült und auf 80 °C aufgeheizt. Zunächst 153 g Ammoniumperoxodisulfat in 500 g demineralisiertem Wasser gelöst und dann 93,87 g 38 %-ige Natriumhydrogensulfit-Lösung wurden hinzugegeben. Die Reaktionsmischung wurde drei Stunden bei 80 °C gerührt. Anschließend wurde die fertige Dispersion mit 3 % 30 %-iger wässriger Wasserstoffperoxidlösung bezüglich Gesamtmenge des Ansatzes versetzt und für 2-3 Stunden stehengelassen und auf Raumtemperatur abgekühlt. Die Reinigung der Partikel erfolgte durch mehrmaliges (mindestens dreimal) Waschen mit demineralisiertem Wasser. Der Raektionsansatz wurde über eine Filternutsche abgesaugt, mit Wasser aufgeschlämmt und das Wasser wiederum abgesaugt. Die Trocknung erfolgte im Trockenschrank bei 50-65 °C bei Normaldruck - wenn nötig zusätzlich unter Vakuum bei 40 °C - bis zur Gewichtskonstanz.

### Beispiel 2 und Vergleichsbeispiel V2:

Es wurden die in Tabelle 1 angegebenen Formulierungen hergestellt und deren Stabilität, Erscheinungsbild und Hautgefühl bewertet.

**Tabelle 1: Formulierungen und Ergebnisse von Beispiel 2 und Vergleichsbeispiel V2, Öl-in-Wasser Pflegecreme**

| | **Beispiel** | **2** | **V2** |
|---|---|---|---|
| **A** | TEGO^{®} Care 165 (Evonik Goldschmidt GmbH) (Glycerylstearat; PEG-100 Stearat) | 6,0 % | 6,0 % |
| | Stearylalkohol | 3,0 % | 3,0 % |
| | Mineralöl | 4,0 % | 4,0 % |
| | Ethylhexylpalmitat | 4,0 % | 4,0 % |
| **B** | Glycerin | 3,0 % | 3,0 % |
| | Wasser | 75,0 % | 80,0 % |
| **C** | Siliconmethacrylat-Partikel aus Bsp. 1 | 5,0 % | |
| **Z** | Methylparaben, Ethylparaben, Methylisothiazolinon, Parfum | q.a. | q.a. |
| | | | |
| | Stabilität | Gut | Gut |
| | Erscheinungsbild | Weiß, homogen | Weiß, homogen |
| | Hautgefühl | Samtig-seidig, glatt; nicht stumpf | Wachsig, stumpf |

### Beispiel 3 und Vergleichsbeispiel V3: kalt hergestellte Öl-in-Wasser Körperpflegelotionen

Es wurden die in Tabelle 2 angegebenen Formulierungen hergestellt und deren Stabilität, Erscheinungsbild und Hautgefühl bewertet.

**Tabelle 2: Formulierung und Ergebnisse der in Beispiel 3 kalt hergestellten Öl-in-Wasser Körperpflegelotion:**

| | **Beispiel** | **3** |
|---|---|---|
| **A** | TEGO^{®} Care LTP (Evonik Goldschmidt GmbH) Sorbitanlaurat; Polyglyceryl-4-laurat; Dilaurylcitrat) | 1,5 % |
| | Cyclopentasiloxan | 10,0 % |
| | Isohexadecan | 3,5 % |
| | Ethylhexylpalmitat | 1,1 % |
| | TEGO^{®} Carbomer 140 (Evonik Degussa GmbH) | 0,15 % |
| | TEGO^{®} Carbomer 141 (Evonik Degussa GmbH) | 0,15 % |
| | Xanthan | 0,1 % |
| **B** | Glycerin | 3,0 % |
| | Wasser | 79,6 % |
| **C** | NaOH (10%ige Lösung) | 0,90 % |
| **D** | Siliconmethacrylat-Partikel aus Bsp. 1 | 5,0 % |
| **Z** | Phenoxyethanol, Benzoesäure, Dehydroessigsäure, Ethylhexylglycerin, Polyaminopropylbiguanid, Parfum | q.a. |
| | | |
| | Stabilität | Gut |
| | Erscheinungsbild | Weiß, homogen |
| | Hautgefühl | Leicht; samtig; glatt |

### Beispiel 4 und Vergleichsbeispiel V4: Wasser-in-Öl Foundation

Es wurden die in Tabelle 3 angegebenen Formulierungen hergestellt und deren Stabilität, Erscheinungsbild und Hautgefühl bewertet.

**Tabelle 3: Wasser-in-Öl Foundation gemäß Beispiel 4 und Vergleichsbeispiel V4:**

| | **Beispiel** | **4** | **V4** |
|---|---|---|---|
| A | ABIL^{®} EM 90 (Evonik Goldschmidt GmbH) (Cetyl PEG/PPG-10/1 Dimethicone) | 3,0 % | 3,0 % |
| | Diethylhexylcarbonat | 10,0 % | 10,0 % |
| | Cyclopentasiloxan | 7,6 % | 7,6 % |
| | Ethylhexylpalmitat | 3,4 % | 3,4 % |
| | Eisenoxid | 1,8 % | 1,8 % |
| | Titandioxid | 7,2 % | 7,2 % |
| | Talkum | 2,0 % | 2,0 % |
| | Siliconmethacrylat-Partikel aus Bsp. 1 | 2,5 % | |
| B | NaCl | 1,0 % | 1,0 % |
| | Glycerin | 2,0 % | 2,0 % |
| | Wasser | 65,5 % | 68,0 % |
| Z | Phenoxyethanol; Methylparaben; Ethylparaben, Butylparaben; Propylparaben, Isobutylparaben, Parfum | q.a. | q.a. |
| | | | |
| | Stabilität | Gut | Gut |
| | Erscheinungsbild | Homogen, bräunlich | Homogen, bräunlich |
| | Hautgefühl | Glatt, nicht stumpf, samtig | Etwas trocken und stumpf |

### Beispiele 5 und V5: Beispiel für Geruchsentwicklung beim Einsatz von Partikeln aus Siliconacrylat TEGO® RC 726.

Es wurden die in Tabelle 4 angegebenen Formulierungen hergestellt und deren Stabilität, Erscheinungsbild und Hautgefühl bewertet.

**Tabelle 4: Öl-in-Wasser Sonnenschutzlotion gemäß Beispiele 5 und V5.**

| | **Beispiel** | **5** | **V5** |
|---|---|---|---|
| **A** | AXOL® C 62 (Evonik Goldschmidt GmbH) (Glycerylstearatcitrat) | 2,00% | 2,00% |
| | Cetearylalkohol | 1,00% | 1,00% |
| | C₁₂₋₁₅Alkylbenzoat | 8,00% | 8,00% |
| | Triisostearin | 1,00% | 1,00% |
| | Diethylhexylcarbonat | 2,75% | 2,75% |
| | Tocopherylacetat | 0,50% | 0,50% |
| | Xanthan | 0,40% | 0,40% |
| | Ethylhexylmethoxycinnamat | 7,00% | 7,00% |
| | Butylmethoxydibenzoylmethan | 3,00% | 3,00% |
| | TEGO® Sun T 805 (Evonik Goldschmidt GmbH) (Titandioxid; Trimethoxy-caprylylsilan) | 2,25% | 2,25% |
| | Siliconacrylat-Partikel aus TEGO® RC 726 (s. DE 200710058713) | | 2,50% |
| | Siliconmethacrylat-Partikel aus Bsp. 1 | 2,50% | |
| **B** | Glycerin | 2,00% | 2,00% |
| | Wasser | 67,60% | 67,60% |
| **Z** | Phenoxyethanol, Ethylhexylglycerin, Parfum | q.a. | q.a. |
| | | | |
| | Stabilität | Gut | Gut |
| | Erscheinungsbild | Weiß, homogen | Weiß, homogen |
| | Hautgefühl | Pflegend, glatt, samtig | Pflegend, glatt, samtig |
| | Geruch nach 2 Wochen Lagerung bei Raumtemperatur | kein Geruch | Deutlicher Geruch nach Acrylsäure und Acrylatverbindungen |

### Vergleichsbeispiel V6: Trockene Lagerung von Siliconacrylatpartikeln aus TEGO® RC 726

Die trockenen Partikel wurden in einem Schraubglas mehrere Wochen bei Raumtemperatur gelagert. Nach vier Wochen war ein schwacher Geruch nach Acrylsäure wahrnehmbar, der sich in den kommenden Wochen noch intensivierte. Die Anwendungsbeispiele zeigen, dass die erfindungsgemäßen Siliconmethacrylat-Partikel in kosmetische Formulierungen eingearbeitet werden können. Durch die Verwendung dieser Partikel werden die sensorischen Eigenschaften von kosmetischen Formulierungen deutlich verbessert, ohne dass sich Stabilität und das Erscheinungsbild der Beipielemulsionen verschlechtern. Insbesondere führt die Einarbeitung der Partikel zu einem samtigeren, seidigeren, weniger trockenen und weniger stumpfen Hautgefühl.

Insbesondere sind die Siliconmethacrylat-Partikel auch geeignet, in Formulierungen zusammen mit Pigmenten eingesetzt zu werden, da sie das üblicherweise etwas stumpfe Hautgefühl pigmenthaltiger Formulierungen deutlich verbessern. Siliconmethacrylatpartikel haben den Vorteil gegenüber Siliconacrylatpartikeln, keinen unangenehmen Geruch nach Acrylsäure oder Acrylatverbindungen zu entwickeln, was letztere besonders für eine Verwendung in kosmetischen Formulierungen ungeeignet macht.

### Beispiele 7 und V7: Emulsionsstabilisierender Effekt der erfindungsgemäßen Siliconmethacrylatpartikel.

Neben den bereits beschriebenen sensorisch erzielbaren Vorteilen zeigte sich auch, dass die erfindungsgemäßen Siliconmethacrylatpartikel die Stabilität kosmetischer Formulierungen verbessern können. Insbesondere zeigte sich dieser Effekt in W/O-Emulsionen. Vergleichsbeispiel V7 zeigt eine W/O Emulsion mit Lichtschutzfiltern, die Ölabscheidung bei Raumtemperatur und in der Wärme zeigt. Zusatz von 2% erfindungsgemäßer Siliconmethacrylatpartikel führt bei insgesamt konstantem Ölphasengehalt zu stabilen Emulsionen.

**Tabelle 5: Wasser-in-Öl Emulsion mit Lichtschutzfiltern gemäß Beispiel 7 und Vergleichsbeispiel V7:**

| | **Beispiel** | **7** | **V7** |
|---|---|---|---|
| A | ABIL^{®} EM 90 (Evonik Goldschmidt GmbH) (Cetyl PEG/PPG-10/1 Dimethicone) | 1,5 % | 1,5 % |
| | ABIL^{®} EM 97 S (Evonik Goldschmidt GmbH) (Bis-PEG/PPG-14/14 Dimethicone; Dimethicone) | 1,5 % | 1,5 % |
| | Diethylhexyl Carbonate | 10,0 % | 11,0 % |
| | Cyclopentasiloxane | 10,0 % | 11,0 % |
| | Butyl Methoxydibenzoylmethane | 1,0 % | 1,0 % |
| | Bis Ethylhexyloxyphenol Methoxyphenyl Triazine | 1,0 % | 1,0 % |
| | Octocrylene | 4,0% | 4,0% |
| | Ethylhexyl Salicylate | 4,0% | 4,0% |
| | Ethylhexyl Triazone | 0,5 % | 0,5 % |
| | Siliconmethacrylat-Partikel aus Bsp. 1 | 2,0% | |
| B | NaCl | 1,0 % | 1,0 % |
| | Glycerin | 2,0% | 2,0 % |
| | Wasser | 60,8 % | 60,8 % |
| Z | Euxyl PE 9010 (Schülke) (Phenoxyethanol; Ethylhexylglycerin | 0,7 % | 0,7 % |
| | | | |
| | Stabilität | Gut | Ölseparation bei RT und 40°C |

### Weitere Formulierungsbeispiele:

### Beispiel 8: O/W Creme gemäß Tabelle 6

**Tabelle 6: O/W Creme gemäß Beispiel 8**

| | |
|---|---|
| Polyglyceryl-3-Methylglucosedistearat | 3,00% |
| Glycerylstearat | 2,00% |
| Cetearylalkohol | 1,00% |
| Ethylhexylstearat | 10,00% |
| Decyloleat | 9,00% |
| Siliconmethacrylat-Partikel aus Bsp. 1 | 1,50% |
| Glycerin | 3,00% |
| Wasser | ad 100% |
| Natriumbenzoat, Kaliumsorbat, Phenoxyethanol, Parfum | q.s. |

### Beispiel 9: Tränklösung für Feuchttücher gemäß Tabelle 7

**Tabelle 7: Tränklösung für Feuchttücher gemäß Beispiel 9**

| | |
|---|---|
| TEGO ^{®} Wipe Flex (Ethylhexylstearat, Phenoxyethanol, Sorbitanlaurat, Polyglyceryl-4-laurat, Dilaurylcitrat) | 5,70% |
| Cyclomethicone | 2,00% |
| Siliconmethacrylat-Partikel aus Bsp. 1 | 2,00% |
| Wasser | ad 100% |
| Glycerin | 3,00% |
| TEGO ^{®} Carbomer 141 | 0.10%. |
| Natriumhydroxid (10% in Wasser) | q.s. |

| | |
|---|---|
| TEGO ^{®} Wipe Flex (Evonik Goldschmidt GmbH) TEGO ^{®} Carbomer 141 (Evonik Goldschmidt GmbH) | |

Die Tränklösung kann mit Hilfe üblicher Tränk- oder Sprühprozesse zur Herstellung von kosmetischen Feuchttüchern verwendet werden (z.B. für Babypflege, Make-Up Entferner, Reinigungstücher). Dazu werden typischerweise Vliesstoffe (non-woven) verwendet, die in der Regel Fasern von Polyolefinen, Polyestern, Cellulosen, Rayon, Polyamiden, Polyesteramiden oder Polyvinylalkoholen aufweisen oder aus diesen bestehen oder die aus gemischten Fasern dieser Komponenten aufgebaut sind.

### Beispiel 10: Make-Up Powder Foundation gemäß Tabelle 8

**Tabelle 8: Make-Up Powder Foundation gemäß Beispiel 10:**

| | |
|---|---|
| Zinkstearat | 3,00% |
| Glimmer | 40,00% |
| Talkum | 24,00% |
| Eisenoxid | 5,00% |
| Siliconmethacrylat-Partikel aus Bsp. 1 | 10,00% |
| Titandioxid | 8,00% |
| Cetylethylhexanoat | 2,00% |
| Squalan | 3,00% |
| Cetearylethylhexanoat | 2,00%. |
| Mineralöl (30 mPas) | 2,00% |
| PEG/PPG-4/12 Dimethicone | 1,00% |
| Aluminium Stärke Octenylsuccinat | q.s. |
| Eisenoxid | q.s. |
| Parfum | q.s. |

### Beispiel 11: Make-Up Foundation gemäß Tabelle 9

**Tabelle 9: Make-Up Foundation gemäß Beispiel 11**

| | |
|---|---|
| Phenyltrimethicone | 14,00% |
| Ethylhexylpalmitat | 14,60% |
| Cetylethylhexanoat | 5,00% |
| Carnaubawachs | 4,70% |
| Stearoxydimethicone | 4,00% |
| PVP/Eicosene Copolymer | 1,00% |
| Cetylstearylheptanoat | 2,85% |
| Covabead LH 85, Polymethylmethacrylat Partikel | 3,00% |
| Siliciumdioxid | 0,25%. |
| Zinkoxid | 7,00% |
| Nylon-12 | 2,00% |
| Talc Covasil 4.05 | 9,50% |
| Acrylat Copolymer | 2,00% |
| Siliconmethacrylat-Partikel aus Bsp. 1 | 3,00% |
| Aluminium Stärke Octenylsuccinat | 9,50% |
| Eisenoxid | 3,10% |
| Titandioxid (und) Dimethicone | 14,50% |

### Beispiel 12 Lidschattenformulierung gemäß 10

**Tabelle 10: Lidschattenformulierung gemäß Beispiel 12**

| | |
|---|---|
| Cyclomethicone | ad 100% |
| PPG-3 Myristylether | 7,00% |
| *Polyglyceryl-4-Isostearat; Cetyl PEG/PPG-10/1 Dimethicone; Hexyllaurat | 1,00% |
| Dimethicone (20 mPas) | 2,50% |
| Cera Alba | 4,50% |
| Carnaubawachs | 2,00% |
| A-C Coploymer 400 (Ethylen/VA Copolymer) | 2,50% |
| Ozokerit | 5,80% |
| C18-36-Säuretriglycerid | 2,00% |
| Liquiparöl (Isobutylparaben (und) Isopropylparaben (und) Butylparaben) | 0,20% |
| Siliconmethacrylat-Partikel aus Bsp. 1 | 4,00% |
| Titandioxid | 5,00% |
| Chromoxid Grün) | 10,00% |
| Cl 77491 (und) Aluminium Puder (und) Siliciumdioxid | 5,00% |
| Cl 77891 (und) Cl 77288 (und) Glimmer | 10,00% |

| | |
|---|---|
| *ABIL ^{®} WE 09 (Evonik Goldschmidt GmbH) | |

### Beispiel 13: O/W Sonnenschutzlotion gemäß Tabelle 11

**Tabelle 11: O/W Sonnenschutzlotion gemäß Beispiel 13**

| | |
|---|---|
| Glycerylstearatcitrat | 3,00% |
| Cetearylalkohol | 1,00% |
| Cetyldimethicone | 0,20% |
| C₁₂-C₁₅ Alkyl Benzoat | 4,80% |
| Triisostearin | 1,00% |
| Diethylhexylcarbonat | 6,00% |
| *Titandioxid; Trimethoxycaprylylsilan | 3,00% |
| Tocopherylacetat | 0,50% |
| Ethylhexylmethoxycinnamat | 5,00%. |
| Butylmethoxydibenzoylmethan | 2,50% |
| Carbomer | 0,20% |
| Xanthan | 0,40% |
| Natriumcarboxymethylbetaglucan | 0,10% |
| Glycerin | 2,00% |
| Wasser | ad 100% |
| Siliconmethacrylat-Partikel aus Bsp. 1 | 1.50% |
| Natriumhydroxid (10% in Wasser) | q.s. |
| Parfum | q.s. |

| | |
|---|---|
| *Tego® Sun T 805 (Evonik Goldschmidt GmbH) | |

### Beispiel 14 AP/Deo Roll-On gemäß Tabelle 12:

**Tabelle 12: Formulierung gemäß Beispiel 14**

| | |
|---|---|
| Steareth-2 | 2,20% |
| Steareth-20 | 1,00% |
| Cetearylethylhexanoat | 2,00% |
| PPG-11 Stearylether | 2,00% |
| Dimethicone | 0,50% |
| Polyglyceryl 3-Caprylat | 0,50% |
| Aluminiumchlorohydrat | 5,00% |
| Wasser | ad 100% |
| Glycerin | 3,00% |
| Siliconmethacrylat-Partikel aus Bsp. 1 | 0,30%. |
| Parfum | q.s. |
| Citronensäure (50% in Wasser) | q.s. |
| Phenoxyethanol, Ethylhexylglycerin | q.s. |

### Beispiel 15 Lippenstiftformulierung gemäß Tabelle 13:

**Tabelle 13: Formulierung gemäß Beispiel 15**

| | |
|---|---|
| Cyclopentasiloxan | 34,00% |
| Behenoxydimethicone | 3,00% |
| Stearyldimethicone | 10,00% |
| Polyisobuten | 5,00% |
| Phenyltrimethicone | 8,00% |
| Isododecan | 4,00% |
| Bis-Diglyceryl Polyacyladipat-2 | 4,00% |
| Ceresin | 24,00% |
| Titandioxid | 1,00% |
| Karminrot | 1,00% |
| D&C Red Nr. 7 | 3,00% |
| Polyethylen | 1,00% |
| Siliconmethacrylat-Partikel aus Bsp. 1 | 1,00% |
| Aluminium Stärke Octenylsuccinat & Lauroyllysin | 1,00% |

### Beispiel 16 Mascaraformulierung gemäß Tabelle 14:

**Tabelle 14: Formulierung gemäß Beispiel 16**

| | |
|---|---|
| Sucrosestearat | 4,00% |
| Polyglyceryl-3 Methylglucosedistearat | 2,00% |
| Stearylalkohol | 1,00% |
| Cadelillawachs | 5,00% |
| Carnaubawachs | 1,75% |
| Bienenwachs | 4,25% |
| Hydriertes Reiskleiewachs | 5,00% |
| Adipinsäure/Diethylenglycol/Glycerin Crosspolymer | 5,00% |
| Ceramid NP | 0,05% |
| Eisenoxid | 10,00% |
| Siliconmethacrylat-Partikel aus Bsp. 1 | 0,50% |
| Wasser | 49,55% |
| 1,3-Butandiol | 3,00% |
| Triethanolamin | 1,80% |
| Acrylate/Octylacrylamid Copolymer | 5,00% |
| Phenoxyethanol; Methylparaben; Ethylparaben, Butylparaben; Propylparaben, Isobutylparaben | 0,60% |
| Phenoxyethanol | 0,50% |

### Beispiel 17 Make-up Foundation gemäß Tabelle 15:

**Tabelle 15: Formulierung gemäß Beispiel 17**

| | |
|---|---|
| *Bis-(Glyceryl/Lauryl) Glyceryl/Lauryl Dimethicone | 4,00% |
| **Diethylhexylcarbonat | 3,10% |
| ***Phenoxyethylcaprylat | 3,10% |
| Mineralöl | 6,30% |
| Titandioxid | 4,00% |
| Eisenoxid | 2,50% |
| Talkum | 2,00% |
| Dicaprylylcarbonat (und) Stearalkonium Hectorit (und) Propylencarbonate | 2,00% |
| Siliconmethacrylat-Partikel aus Bsp. 1 | 1,00% |
| ****Nylon-1010 | 1,00% |
| Dimethicone; Dimethicone/Vinyl Dimethicone Crosspolymer | 4,00% |
| Glycerin | 4,00% |
| Magnesiumsulfat Heptahydrat | 1,50% |
| Wasser | 60,80% |
| Methylparaben; Ethylparaben; Propylparaben; n-Propylparaben; Phenoxtol | 0,70% |

| | |
|---|---|
| *ABIL ^{®} EM 120 (Evonik Industries AG) **TEGOSOFT^{®} DEC (Evonik Industries AG) ***TEGOSOFT^{®} XC (Evonik Industries AG) ****TEGOLON^{®} ECO 10-10 (Evonik Industries AG) | |

### Beispiel 18 Duschgel-Formulierung gemäß Tabelle 16:

**Tabelle 16: Formulierung gemäß Beispiel 18**

| | |
|---|---|
| *Acrylate/C10-30 Alkyl Acrylat Crosspolymer | 1,60% |
| Wasser | 63,30% |
| Natriumlaurylsulfat | 21,40% |
| **Cocamidopropylbetain | 5,30% |
| ***Polyglyceryl-3 Caprat | 0,50% |
| Siliconmethacrylat-Partikel aus Bsp. 1 | 2,00% |
| Natriumhydroxid (10% in Wasser) | q.s. |
| Phenoxyethanol; Methylisothiazolinon | q.s. |
| Parfum | q.s. |

| | |
|---|---|
| *TEGO^{®} Carbomer 341 ER (Evonik Industries AG) **TEGO^{®} Betain F 50 (Evonik Industries AG) ***TEGOSOFT^{®} PC 31 (Evonik Industries AG) | |

### Beispiel 19 Conditioner-Formulierung gemäß Tabelle 17:

**Tabelle 17: Formulierung gemäß Beispiel 19**

| | |
|---|---|
| Wasser | 89,00% |
| *Cetrimoniumchlorid | 2,00% |
| **Behentrimoniumchlorid | 2,00% |
| ***Cyclopentasiloxan; Dimethiconol | 1,00% |
| Siliconmethacrylat-Partikel aus Bsp. 1 | 0,50% |
| ****Cetearylalkohol | 5,00% |
| Phenoxyethanol; Methylisothiazolinon | q.s. |
| Parfum | q.s. |

| | |
|---|---|
| *VARISOFT^{®} 300 (Evonik Industries AG) **VARISOFT^{®} BT 85 (Evonik Industries AG) ***ABIL^{®} OSW 5 (Evonik Industries AG) ****TEGO^{®} Alkanol 1618 (Evonik Industries AG) | |

### Beispiel 20 2-in-1-Shampoo-Formulierung gemäß Tabelle 18:

**Tabelle 18: Formulierung gemäß Beispiel 20**

| | |
|---|---|
| Natriumlaurylsulfat | 60,00% |
| Natriumcumolsulfonat | 3,00% |
| Wasser | 21,25% |
| *Cocamidopropylbetain | 8,00% |
| **Cocamid MEA | 1,50% |
| ***Glycoldistearat | 1,50% |
| ****Cetylalkohol | 0,50% |
| Dimethicone (1000 mPa*s) | 1,50% |
| Siliconmethacrylat-Partikel aus Bsp. 1 | 1,00% |
| *****Nylon-1010 | 0,50% |
| Xanthan Gum | 0,75% |
| Phenoxyethanol; Methylisothiazolinon | q.s. |
| Parfum | q.s. |

| | |
|---|---|
| *TEGO^{®} Betain F 50 (Evonik Industries AG) **REWOMID^{®} C 212 (Evonik Industries AG) ***TEGIN^{®} G 100 (Evonik Industries AG) ****TEGO^{®} Alkanol 16 (Evonik Industries AG) *****TEGOLON^{®} ECO 10-10 (Evonik Industries AG) | |

### Beispiel 21 Styling Wax-Formulierung gemäß Tabelle 19:

**Tabelle 19: Formulierung gemäß Beispiel 21**

| | |
|---|---|
| Wasser | 48,00% |
| Propylenglykol | 2,00% |
| Glycerin | 11,00% |
| *Methoxy PEG/PPG-7/3 Aminopropyldimethicone | 0,50% |
| **Isosteareth-20 | 14,50% |
| ***Laureth-4 | 10,00% |
| Paraffinum Perliquidum | 6,00% |
| ****C12-15 Alkyl Benzoate | 6,00% |
| Siliconmethacrylat-Partikel aus Bsp. 1 | 1,00% |
| Phenoxyethanol; Methylisothiazolinon | q.s. |
| Parfum | q.s. |

| | |
|---|---|
| *ABIL^{®} Soft AF 100 (Evonik Industries AG) **REWODERM^{®} 66 E 20 (Evonik Industries AG) ***TEGO^{®} Alkanol L 4 (Evonik Industries AG) ****TEGOSOFT^{®} TN (Evonik Industries AG) | |

### Beispiel 22 Leave-in-Conditioner-Formulierung gemäß Tabelle 20:

**Tabelle 20: Formulierung gemäß Beispiel 22**

| | |
|---|---|
| *Ceteareth-25 | 4,00% |
| **Cyclopentasiloxan; Dimethiconol | 16,00% |
| ***Methoxy PEG/PPG-7/3 Aminopropyldimethicone | 1,00% |
| Siliconmethacrylat-Partikel aus Bsp. 1 | 3,00% |
| ****Laureth-4 | 0,50% |
| *****Carbomer | 0,50% |
| Wasser | 67,00% |
| Propylenglykol | 5,00% |
| Natriumhydroxid (10 % in Wasser) | ad pH 5-6 |
| Parfum | q.s. |
| Phenoxyethanol; Methylisothiazolinon | q.s. |

| | |
|---|---|
| *TEGINACID^{®} C (Evonik Industries AG) **ABIL^{®} OSW 5 (Evonik Industries AG) ***ABIL^{®} SoftAF 100 (Evonik Industries AG) ****TEGO^{®} Alkanol L 4 (Evonik Industries AG) *****TEGO^{®} Carbomer 140 (Evonik Industries AG) | |

## Patentansprüche

1. Kosmetische Zusammensetzung enthaltend Feststoffpartikel, **dadurch gekennzeichnet, dass** die Zusammensetzung als Feststoffpartikel zumindest Siliconmethacrylat-Partikel aufweist, die erhältlich sind durch die Schritte
a) Erzeugen einer Emulsion aus Wasser und einer organischen Phase, wobei die organische Phase, end- und/oder seitenständig mit Methacrylat-Gruppen modifizierte Organopolysiloxane nach der allgemeinen Formel (I) oder deren Gemische 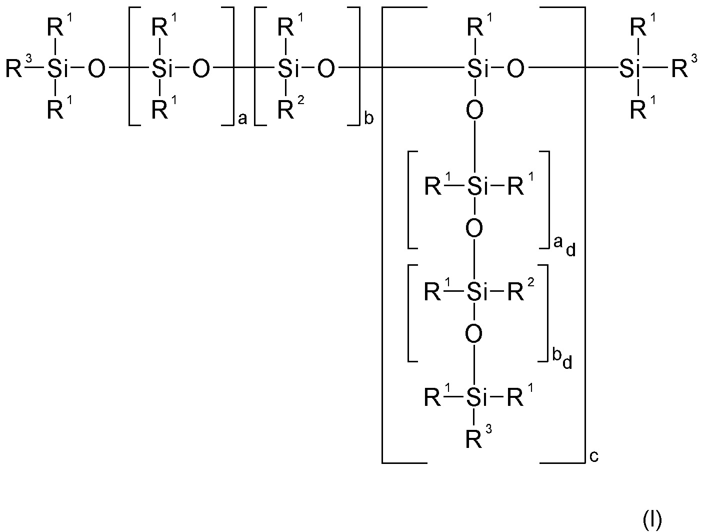 mit
R¹ = gleiche oder verschiedene Reste, ausgewählt aus linearen oder verzweigten, gesättigten, ein- oder mehrfach ungesättigten, gegebenenfalls eine oder mehrere Ether- oder Esterbrücken enthaltende lineare, cyclische oder verzweigte Alkyl-, Alkoxy-, Polyalkoxy-, Hydroxyalkyl-, Hydroxyalkoxy-, Alkenyl-, Aryl-, Aryloxy-, Hydroxyaryl-, Hydroxyaryloxy-, Alkaryl-, Alkaryloxy-, Hydroxyalkaryl-, Hydroxyalkaryloxy-, Aralkyl-, Aralkoxy-, Hydroxyaralkyl- oder Hydroxyaralkoxy-Resten mit 1 bis 20 C-Atomen, vorzugsweise Methyl-Rest,
R² = gleiche oder verschiedene, an das Si-Atom über eine Si-C-Verknüpfung gebundene, zweiwertige, Kohlenwasserstoffreste, mit 1 bis 20 C-Atomen, an welche mindestens eine Methacrylsäure-Einheiten über eine Esterbindung angebunden ist,
R³ = gleiche oder verschiedene Reste R¹ oder R², vorzugsweise R²,
a = 50 bis 1.000, vorzugsweise 100 bis 210, besonders bevorzugt 140 bis 190
b = 0 bis 15, vorzugsweise 5 bis 10,
c = 0 bis 5, vorzugsweise 0,
a_{d} = 0 bis 1.000,
b_{d} = 0 bis 15,
wobei
der Index d für c > 0 eine ganze Zahl > 0 ist,
mit der Maßgabe, dass wenn b und c = 0 sind, R³ nicht aus der gleichen Gruppe wie R¹ ausgewählt sein darf, enthält, unter Zugabe zumindest eines Emulgators und optional eines oder mehrerer Coemulgatoren, wobei die organische Phase die innere Phase der Emulsion bildet, und
b) Auspolymerisieren der inneren Phase in Gegenwart eines Radikalstarters, welcher der äußeren Phase (wässrigen Phase) in einer Konzentration von 0,1 bis 40 Gew.-% bezogen auf die innere Phase zugefügt wird.

2. Zusammensetzung nach Anspruch 1, **dadurch gekennzeichnet, dass** die Zusammensetzung Siliconmethacrylat-Partikel aufweist, die ein Maximum der Partikelgrößenverteilung im Bereich von 1 bis 50 µm aufweisen.

3. Zusammensetzung nach Anspruch 1 oder 2, **dadurch gekennzeichnet, dass** der Anteil der Siliconmethacrylat-Partikel an der Gesamtzusammensetzung von 0,1 bis 10 Gew.-% beträgt.

4. Zusammensetzung nach zumindest einem der Ansprüche 1 bis 3, **dadurch gekennzeichnet, dass** die Zusammensetzung von 0,1 bis 20 Gew.-% bezogen auf die Gesamtzusammensetzung an Komponenten aufweist, die UV-Strahlung absorbieren oder ausfiltern.

5. Zusammensetzung nach zumindest einem der Ansprüche 1 bis 4, **dadurch gekennzeichnet, dass** die Zusammensetzung von 0,1 bis 15 Gew.-% an kosmetischen Partikeln aufweist, die von den in Anspruch 1 genannten Siliconmethacrylat-Partikeln verschieden sind.

6. Zusammensetzung nach zumindest einem der Ansprüche 1 bis 5, **dadurch gekennzeichnet, dass** die Zusammensetzung von 0,1 bis 20 Gew.-% an siliziumorganischen Verbindungen aufweist.

7. Zusammensetzung nach zumindest einem der Ansprüche 1 bis 6, **dadurch gekennzeichnet, dass** solche Siliconmethacrylat-Partikel enthalten sind, die durch Erzeugen einer festkörperstabilisierte Emulsion in Schritt a) erhalten wurden, wobei als Emulgatoren partikuläre Emulgatoren, die ausgewählt sind aus der Gruppe der Metalloxide, Mischoxide, Nitride, Hydroxide, Carbonate, Silikate, Siliconharze, Silicone und/oder organischen Polymere, die zumindest (teil-)hydrophobiert sind mit mindestens einer Verbindung aus der Gruppe der Silane, Siloxane, quaternären Ammoniumverbindungen, kationischen Polymere und Fettsäuren oder deren Anionen, eingesetzt werden.

8. Zusammensetzung nach mindestens einem der Ansprüche 1 bis 7, **dadurch gekennzeichnet, dass** mehr als 95 mol-% der Reste R¹ in Formel (I) Methylgruppen sind.

9. Zusammensetzung nach mindestens einem der Ansprüche 1 bis 8, **dadurch gekennzeichnet, dass** R² in der allgemeinen Formel(I) aus der Gruppe der Reste 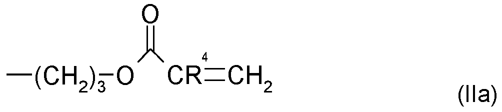 und 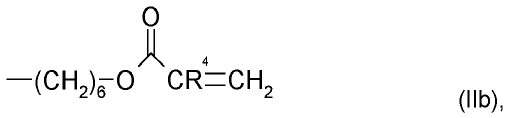 ausgewählt ist,
wobei R⁴ eine Methylgruppe ist.

10. Zusammensetzung nach mindestens einem der Ansprüche 1 bis 9, **dadurch gekennzeichnet, dass** solche Siliconmethacrylat-Partikel eingesetzt werden, bei deren Herstellung Siliconmethacrylate der Formel (I) eingesetzt werden, bei denen a einen Wert von 150 bis 210 und b einen Wert von 3 bis 9 einnimmt und c = 0 ist.

11. Verwendung von Siliconmethacrylat-Partikel, die erhältlich sind durch die Schritte
a) Erzeugen einer Emulsion aus Wasser und einer organischen Phase, wobei die organische Phase, end- und/oder seitenständig mit Methacrylat-Gruppen modifizierte Organopolysiloxane nach der allgemeinen Formel (I) oder deren Gemische 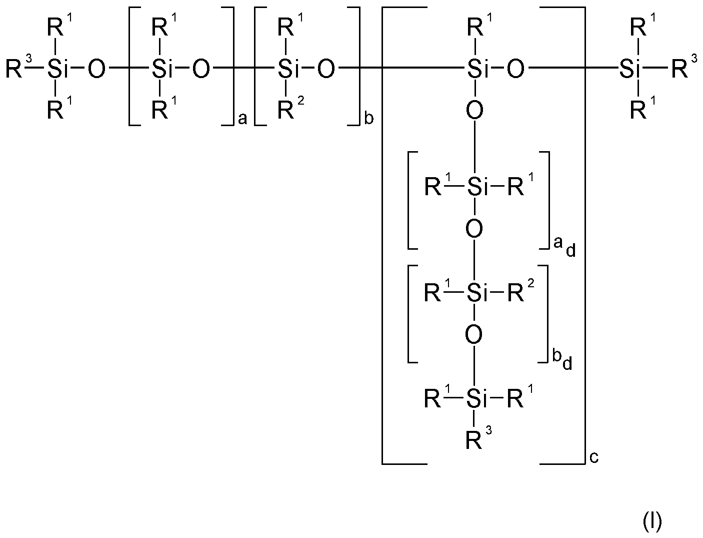 mit
R¹ = gleiche oder verschiedene Reste, ausgewählt aus linearen oder verzweigten, gesättigten, ein- oder mehrfach ungesättigten, gegebenenfalls eine oder mehrere Ether- oder Esterbrücken enthaltende lineare, cyclische oder verzweigte Alkyl-, Alkoxy-, Polyalkoxy-, Hydroxyalkyl-, Hydroxyalkoxy-, Alkenyl-, Aryl-, Aryloxy-, Hydroxyaryl-, Hydroxyaryloxy-, Alkaryl-, Alkaryloxy-, Hydroxyalkaryl-, Hydroxyalkaryloxy-, Aralkyl-, Aralkoxy-, Hydroxyaralkyl- oder Hydroxyaralkoxy-Resten mit 1 bis 20 C-Atomen, vorzugsweise Methyl-Rest,
R² = gleiche oder verschiedene, an das Si-Atom über eine Si-C-Verknüpfung gebundene, zweiwertige, Kohlenwasserstoffreste, mit 1 bis 20 C-Atomen, an welche mindestens eine Methacrylsäure-Einheiten über eine Esterbindung angebunden ist,
R³ = gleiche oder verschiedene Reste R¹ oder R², vorzugsweise R²,
a = 50 bis 1.000, vorzugsweise 100 bis 210, besonders bevorzugt 140 bis 190
b = 0 bis 15, vorzugsweise 5 bis 10,
c = 0 bis 5, vorzugsweise 0,
a_{d} = 0 bis 1.000,
b_{d} = 0 bis 15,
wobei
der Index d für c > 0 eine ganze Zahl > 0 ist,
mit der Maßgabe, dass wenn b und c = 0 sind, R³ nicht aus der gleichen Gruppe wie R¹ ausgewählt sein darf, enthält, unter Zugabe zumindest eines Emulgators und optional eines oder mehrerer Coemulgatoren, wobei
die organische Phase die innere Phase der Emulsion bildet, und
b) Auspolymerisieren der inneren Phase in Gegenwart eines Radikalstarters, welcher der äußeren Phase (wässrigen Phase) in einer Konzentration von 0,1 bis 40 Gew.-% bezogen auf die innere Phase zugefügt wird,
zur Herstellung kosmetischer Zusammensetzungen enthaltend Feststoffpartikel.

12. Verwendung nach Anspruch 11, **dadurch gekennzeichnet, dass** Siliconmethacrylat-Partikel eingesetzt werden, die ein Maximum der Partikelgrößenverteilung im Bereich von 1 bis 50 µm aufweisen.

13. Verwendung nach Anspruch 11 oder 12, **dadurch gekennzeichnet, dass** solche Siliconmethacrylat-Partikel eingesetzt werden, die durch Erzeugen einer festkörperstabilisierte Emulsion in Schritt a) erhalten wurden, wobei als Emulgatoren partikuläre Emulgatoren, die ausgewählt sind aus der Gruppe der Metalloxide, Mischoxide, Nitride, Hydroxide, Carbonate, Silikate, Siliconharze, Silicone und/oder organischen Polymere, die zumindest (teil-)hydrophobiert sind mit mindestens einer Verbindung aus der Gruppe der Silane, Siloxane, quaternären Ammoniumverbindungen, kationischen Polymere und Fettsäuren oder deren Anionen, eingesetzt werden.

14. Verwendung nach mindestens einem der Ansprüche 11 bis 13, **dadurch gekennzeichnet, dass** mehr als 95 mol-% der Reste R¹ in Formel (I) Methylgruppen sind.

15. Verwendung nach mindestens einem der Ansprüche 11 bis 14, **dadurch gekennzeichnet, dass** R² in der allgemeinen Formel(I) aus der Gruppe der Reste 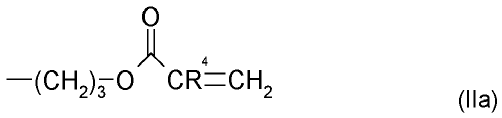 und 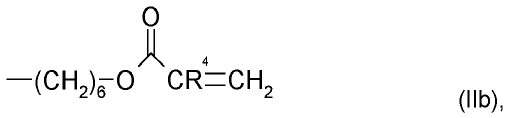 ausgewählt ist,
wobei R⁴ eine Methylgruppe ist.

16. Verwendung nach mindestens einem der Ansprüche 11 bis 15, **dadurch gekennzeichnet, dass** solche Siliconmethacrylat-Partikel eingesetzt werden, bei deren Herstellung Siliconmethacrylate der Formel (I) eingesetzt werden, bei denen a einen Wert von 150 bis 210 und b einen Wert von 3 bis 9 einnimmt und c = 0 ist.

## Claims

1. Cosmetic composition comprising solid particles **characterized in that** the composition has, as solid particles, at least silicone methacrylate particles which are obtainable by the steps
a) producing an emulsion of water and an organic phase, where the organic phase comprises organopolysiloxanes modified in the terminal and/or lateral position with methacrylate groups according to the general formula (I) or mixtures thereof 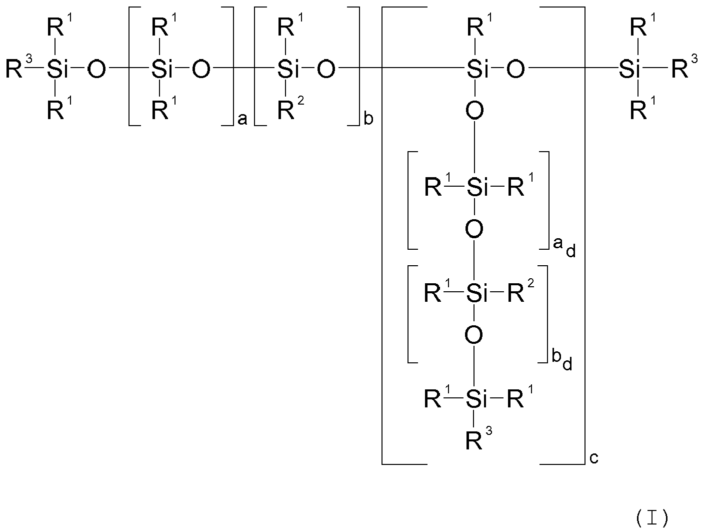 where
R¹ = identical or different radicals, selected from linear or branched, saturated, mono- or polyunsaturated, linear, cyclic or branched alkyl, alkoxy, polyalkoxy, hydroxyalkyl, hydroxyalkoxy, alkenyl, aryl, aryloxy, hydroxyaryl, hydroxyaryloxy, alkaryl, alkaryloxy, hydroxyalkaryl, hydroxyalkaryloxy, aralkyl, aralkoxy, hydroxyaralkyl or hydroxyaralkoxy radicals having 1 to 20 carbon atoms, optionally containing one or more ether or ester bridges, preferably methyl radical,
R² = identical or different divalent hydrocarbon radicals having 1 to 20 carbon atoms bonded to the Si atom via an Si-C linkage and to which at least one methacrylic acid unit is attached via an ester bond,
R³ = identical or different radicals R¹ or R², preferably R²,
a = 50 to 1000, preferably 100 to 210, particularly preferably 140 to 190,
b = 0 to 15, preferably 5 to 10,
c = 0 to 5, preferably 0,
a_{d} = 0 to 1000,
b_{d} = 0 to 15,
where
the index d when c > 0 is an integer > 0,
with the proviso that when b and c = 0, R³ must not be selected from the same group as R¹, with the addition of at least one emulsifier and optionally one or more coemulsifiers,
where the organic phase forms the internal phase of the emulsion, and
b) fully polymerizing the internal phase in the presence of a radical initiator, which is added to the external phase (aqueous phase) in a concentration of from 0.1 to 40% by weight based on the internal phase.

2. Composition according to Claim 1, **characterized in that** the composition has silicone methacrylate particles which have a maximum of the particle size distribution in the range from 1 to 50 µm.

3. Composition according to Claim 1 or 2, **characterized in that** the fraction of the silicone methacrylate particles in the overall composition is from 0.1 to 10% by weight.

4. Composition according to at least one of Claims 1 to 3, **characterized in that** the composition has from 0.1 to 20% by weight based on the total composition, of components which absorb or filter out UV radiation.

5. Composition according to at least one of Claims 1 to 4, **characterized in that** the composition has from 0.1 to 15% by weight of cosmetic particles which are different from the silicone methacrylate particles specified in Claim 1.

6. Composition according to at least one of Claims 1 to 5, **characterized in that** the composition has from 0.1 to 20% by weight of organosilicon compounds.

7. Composition according to at least one of Claims 1 to 6, **characterized in that** those silicone methacrylate particles are present which have been obtained by producing a solids-stabilized emulsion in step a), where the emulsifiers used are particulate emulsifiers which are selected from the group of metal oxides, mixed oxides, nitrides, hydroxides, carbonates, silicates, silicone resins, silicones and/or organic polymers which are at least (partially) hydrophobicized and with at least one compound from the group of silanes, siloxanes, quaternary ammonium compounds, cationic polymers and fatty acids or anions thereof.

8. Composition according to at least one of Claims 1 to 7, **characterized in that**, more than 95 mol% of the radicals R¹ in formula (I) are methyl groups.

9. Composition according to at least one of Claims 1 to 8, **characterized in that** R² in the general formula (I) is selected from the group of the radicals 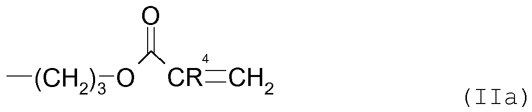 and 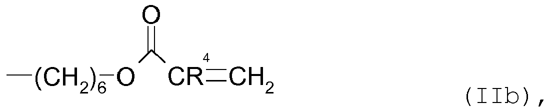 where R⁴ is a methyl group.

10. Composition according to at least one of Claims 1 to 9, **characterized in that** those silicone methacrylate particles are used, in the preparation of which silicone methacrylates of the formula (I) are used in which a assumes a value from 150 to 210 and b assumes a value from 3 to 9 and c = 0.

11. Use of silicone methacrylate particles which are obtainable by the steps
a) producing an emulsion of water and an organic phase, where the organic phase comprises organopolysiloxanes modified in the terminal and/or lateral position with methacrylate groups according to the general formula (I) or mixtures thereof, 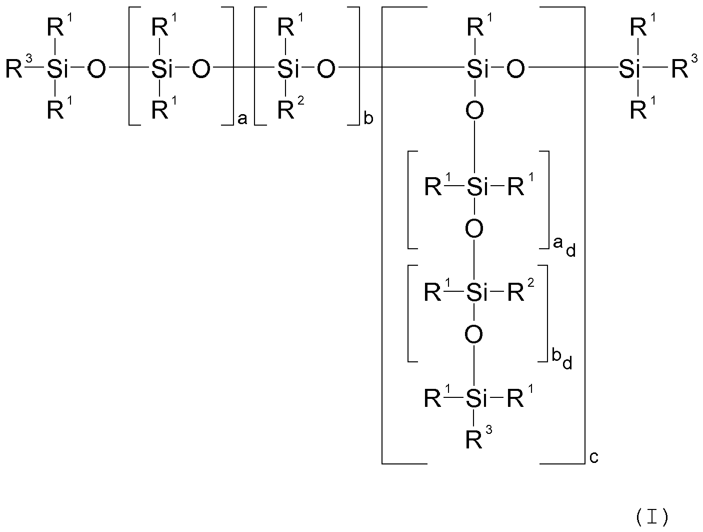 where
R¹ = identical or different radicals, selected from linear or branched, saturated, mono- or polyunsaturated, linear, cyclic or branched alkyl, alkoxy, polyalkoxy, hydroxyalkyl, hydroxyalkoxy, alkenyl, aryl, aryloxy, hydroxyaryl, hydroxyaryloxy, alkaryl, alkaryloxy, hydroxyalkaryl, hydroxyalkaryloxy, aralkyl, aralkoxy, hydroxyaralkyl or hydroxyaralkoxy radicals having 1 to 20 carbon atoms, optionally containing one or more ether or ester bridges, preferably methyl radical,
R² = identical or different divalent hydrocarbon radicals having 1 to 20 carbon atoms bonded to the Si atom via an Si-C linkage and to which at least one methacrylic acid unit is attached via an ester bond,
R³ = identical or different radicals R¹ or R², preferably R²,
a = 50 to 1000, preferably 100 to 210, particularly preferably 140 to 190,
b = 0 to 15, preferably 5 to 10,
c = 0 to 5, preferably 0,
a_{d} = 0 to 1000,
b_{d} = 0 to 15,
where
the index d when c > 0 is an integer > 0,
with the proviso that when b and c = 0, R³ must not be selected from the same group as R¹, with the addition of at least one emulsifier and optionally one or more coemulsifiers,
where the organic phase forms the internal phase of the emulsion, and
b) fully polymerizing the internal phase in the presence of a radical initiator, which is added to the external phase (aqueous phase) in a concentration of from 0.1 to 40% by weight based on the internal phase,
for producing cosmetic compositions comprising solid particles.

12. Use according to Claim 11, **characterized in that** silicone methacrylate particles are used which have a maximum of the particle size distribution in the range from 1 to 50 µm.

13. Use according to Claim 11 or 12, **characterized in that** those silicone methacrylate particles are used which have been obtained by producing a solids-stabilized emulsion in step a), where the emulsifiers used are particulate emulsifiers which are selected from the group of metal oxides, mixed oxides, nitrides, hydroxides, carbonates, silicates, silicone resins, silicones and/or organic polymers, which are at least (partially) hydrophobicized with at least one compound from the group of silanes, siloxanes, quaternary ammonium compounds, cationic polymers and fatty acids or anions thereof.

14. Use according to at least one of Claims 11 to 13, **characterized in that** more than 95 mol% of the radicals R¹ in formula (I) are methyl groups.

15. Use according to at least one of Claims 11 to 14, **characterized in that** R² in the general formula (I) is selected from the group of the radicals 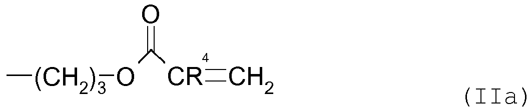 and 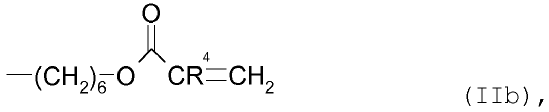 where R⁴ is a methyl group.

16. Use according to at least one of Claims 11 to 15, **characterized in that** those silicone methacrylate particles are used, in the preparation of which silicone methacrylates of the formula (I) are used in which a assumes a value from 150 to 210 and b assumes a value from 3 to 9 and c = 0.

## Revendications

1. Composition cosmétique contenant des particules solides, **caractérisée en ce que** la composition présente, comme particules solides, au moins des particules de méthacrylate de silicone, qui peuvent être obtenues par les étapes consistant à
a) produire une émulsion d'eau et d'une phase organique, la phase organique contenant des organopolysiloxanes modifiés en position terminale et/ou latérale par des groupes méthacrylate selon la formule générale (I) ou leurs mélanges 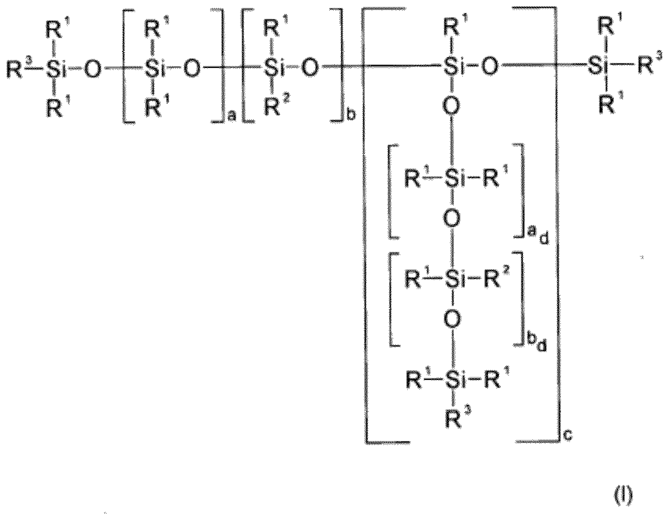 dans laquelle
R¹ = des radicaux identiques ou différents, choisis parmi les radicaux alkyle, alcoxy, polyalcoxy, hydroxyalkyle, hydroxyalcoxy, alcényle, aryle, aryloxy, hydroxyaryle, hydroxyaryloxy, alcaryle, alcaryloxy, hydroxyalcaryle, hydroxyalcaryloxy, aralkyle, aralcoxy, hydroxyaralkyle ou hydroxyaralcoxy, linéaires ou ramifiés, saturés, monoinsaturés ou polyinsaturés, linéaires, cycliques ou ramifiés, contenant le cas échéant un ou plusieurs ponts éther ou ester, comprenant 1 à 20 atomes de carbone, de préférence le radical méthyle,
R² = des radicaux hydrocarbonés identiques ou différents, liés à l'atome Si via une liaison Si-C, divalents, comprenant 1 à 20 atomes de carbone, auxquels est liée au moins une unité d'acide méthacrylique via une liaison ester,
R³ = des radicaux R¹ ou R² identiques ou différents, de préférence R²,
a = 50 à 1000, de préférence 100 à 210 et de manière particulièrement préférée 140 à 190,
b = 0 à 15, de préférence 5 à 10,
c = 0 à 5, de préférence 0,
a_{d} = 0 à 1000,
b_{d} = 0 à 15,
où
l'indice d pour c > 0 est un nombre entier > 0,
à condition que lorsque b et c = 0, R³ ne puisse pas être choisi dans le même groupe que R¹, avec addition d'au moins un émulsifiant et éventuellement d'un ou plusieurs co-émulsifiants, la phase organique formant la phase interne de l'émulsion, et
b) polymériser la phase interne en présence d'un initiateur radicalaire, qui est ajouté à la phase externe (phase aqueuse) en une concentration de 0,1 à 40% en poids par rapport à la phase interne.

2. Composition selon la revendication 1, **caractérisée en ce que** la composition présente des particules de méthacrylate de silicone qui présentent un maximum de la répartition des grosseurs de particule dans la plage de 1 à 50 µm.

3. Composition selon la revendication 1 ou 2, **caractérisée en ce que** la proportion des particules de méthacrylate de silicone par rapport à la composition totale est de 0,1 à 10% en poids.

4. Composition selon au moins l'une quelconque des revendications 1 à 3, **caractérisée en ce que** la composition présente 0,1 à 20% en poids, par rapport à la composition totale, de composants qui absorbent ou éliminent par filtrage le rayonnement UV.

5. Composition selon au moins l'une quelconque des revendications 1 à 4, **caractérisée en ce que** la composition présente 0,1 à 15% en poids de particules cosmétiques qui sont différentes des particules de méthacrylate de silicone mentionnées dans la revendication 1.

6. Composition selon au moins l'une quelconque des revendications 1 à 5, **caractérisée en ce que** la composition présente 0,1 à 20% en poids de composés organosiliciés.

7. Composition selon au moins l'une quelconque des revendications 1 à 6, **caractérisée en ce qu'**elle contient des particules de méthacrylate de silicone qui ont été obtenues par production d'une émulsion stabilisée par des corps solides dans l'étape a), des émulsifiants particulaires, qui sont choisis dans le groupe des oxydes métalliques, des oxydes mixtes, des nitrures, des hydroxydes, des carbonates, des silicates, des résines de silicone, des silicones et/ou des polymères organiques qui sont au moins (partiellement) hydrofugés par au moins un composé du groupe des silanes, des siloxanes, des composés d'ammonium quaternaire, des polymères cationiques et des acides gras ou de leurs anions, étant utilisés comme émulsifiants.

8. Composition selon au moins l'une quelconque des revendications 1 à 7, **caractérisée en ce que** plus de 95% en mole des radicaux R¹ dans la formule (I) sont des groupes méthyle.

9. Composition selon au moins l'une quelconque des revendications 1 à 8, **caractérisée en ce que** R² dans la formule générale (I) est choisi dans le groupe des radicaux 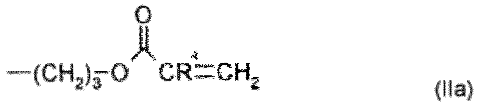 et 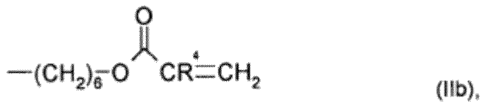 R⁴ représentant un groupe méthyle.

10. Composition selon au moins l'une quelconque des revendications 1 à 9, **caractérisée en ce qu'**on utilise des particules de méthacrylate de silicone lors de la fabrication desquelles des méthacrylates de silicone de formule (I) sont utilisés, dans laquelle a présente une valeur de 150 à 210 et b présente une valeur de 3 à 9 et c = 0.

11. Utilisation de particules de méthacrylate de silicone, qui peuvent être obtenues par les étapes consistant à
a) produire une émulsion d'eau et d'une phase organique, la phase organique contenant des organopolysiloxanes modifiés en position terminale et/ou latérale par des groupes méthacrylate selon la formule générale (I) ou leurs mélanges 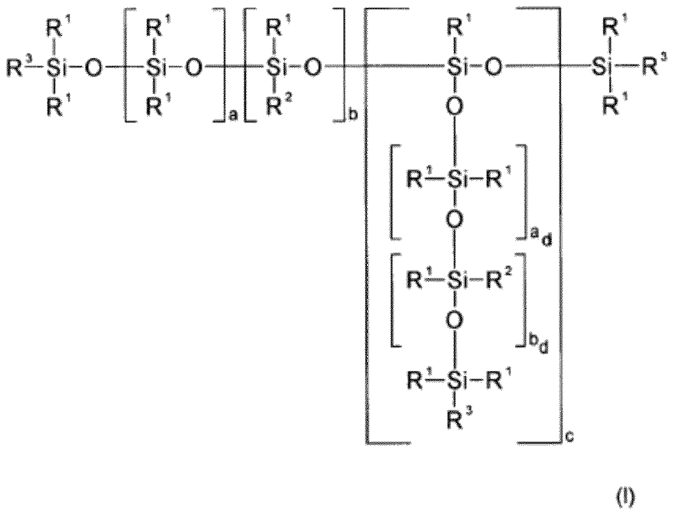 où
R¹ = des radicaux identiques ou différents, choisis parmi les radicaux alkyle, alcoxy, polyalcoxy, hydroxyalkyle, hydroxyalcoxy, alcényle, aryle, aryloxy, hydroxyaryle, hydroxyaryloxy, alcaryle, alcaryloxy, hydroxyalcaryle, hydroxyalcaryloxy, aralkyle, aralcoxy, hydroxyaralkyle ou hydroxyaralcoxy, linéaires ou ramifiés, saturés, monoinsaturés ou polyinsaturés, linéaires, cycliques ou ramifiés, contenant le cas échéant un ou plusieurs ponts éther ou ester, comprenant 1 à 20 atomes de carbone, de préférence le radical méthyle,
R² = des radicaux hydrocarbonés identiques ou différents, liés à l'atome Si via une liaison Si-C, divalents, comprenant 1 à 20 atomes de carbone, auxquels est liée au moins une unité d'acide méthacrylique via une liaison ester,
R³ = des radicaux R¹ ou R² identiques ou différents, de préférence R²,
a = 50 à 1000, de préférence 100 à 210 et de manière particulièrement préférée 140 à 190,
b = 0 à 15, de préférence 5 à 10,
c = 0 à 5, de préférence 0,
a_{d} = 0 à 1000,
b_{d} = 0 à 15,
où
l'indice d pour c > 0 est un nombre entier > 0,
à condition que lorsque b et c = 0, R³ ne puisse pas être choisi dans le même groupe que R¹, avec addition d'au moins un émulsifiant et éventuellement d'un ou plusieurs co-émulsifiants, où
la phase organique forme la phase interne de l'émulsion, et
b) polymériser la phase interne en présence d'un initiateur radicalaire, qui est ajouté à la phase externe (phase aqueuse) en une concentration de 0,1 à 40% en poids par rapport à la phase interne,
pour la production de compositions cosmétiques contenant des particules solides.

12. Utilisation selon la revendication 11, **caractérisée en ce qu'**on utilise des particules de méthacrylate de silicone qui présentent un maximum de la répartition des grosseurs de particule dans la plage de 1 à 50 µm.

13. Utilisation selon la revendication 11 ou 12, **caractérisée en ce qu'**on utilise des particules de méthacrylate de silicone qui ont été obtenues par production d'une émulsion stabilisée par des corps solides dans l'étape a), des émulsifiants particulaires, qui sont choisis dans le groupe des oxydes métalliques, des oxydes mixtes, des nitrures, des hydroxydes, des carbonates, des silicates, des résines de silicone, des silicones et/ou des polymères organiques qui sont au moins (partiellement) hydrofugés par au moins un composé du groupe des silanes, des siloxanes, des composés d'ammonium quaternaire, des polymères cationiques et des acides gras ou de leurs anions, étant utilisés comme émulsifiants.

14. Utilisation selon au moins l'une quelconque des revendications 11 à 13, **caractérisée en ce que** plus de 95% en mole des radicaux R¹ dans la formule (I) sont des groupes méthyle.

15. Utilisation selon au moins l'une quelconque des revendications 11 à 14, **caractérisée en ce que** R² dans la formule générale (I) est choisi dans le groupe des radicaux 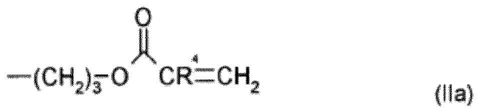 et 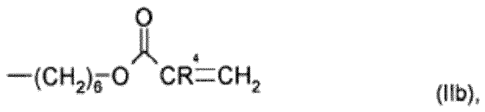 R⁴ représentant un groupe méthyle.

16. Utilisation selon au moins l'une quelconque des revendications 11 à 15, **caractérisée en ce qu'**on utilise des particules de méthacrylate de silicone lors de la fabrication desquelles des méthacrylates de silicone de formule (I) sont utilisés, dans laquelle a présente une valeur de 150 à 210 et b présente une valeur de 3 à 9 et c = 0.
